# EUROPEAN PATENT SPECIFICATION

(11) **EP 3 285 680 B1**
(45) Date of publication and mention of the grant of the patent: **08.08.2018**
(21) Application number: 16727537.9
(22) Date of filing: 23.04.2016
(51) Int. Cl.: A63B 21/02, A63B 21/00, A63B 23/03, A61C 7/36

(54) **INSTRUMENT FOR THE EXERCISE OF THE MUSCLES OF THE MOUTH**
INSTRUMENT ZUM TRAINIEREN DER MUNDMUSKULATUR
INSTRUMENT POUR L'EXERCICE DES MUSCLES DE LA BOUCHE

(30) Priority: 24.04.2015 IT FI20150119
(43) Date of publication of application: 28.02.2018
(73) Proprietor: Ercoli, Tessa, 50143 Florence (IT); Montani, Daniele, 50136 Florence (IT)
(72) Inventor: Ercoli, Tessa, 50143 Florence (IT); Montani, Daniele, 50136 Florence (IT)
(74) Representative: Nesti, Antonio
(86) International application number: PCT/IB2016/000517
(87) International publication number: WO 2016/170417

(56) References cited:
- US-A- 4 280 696
- US-A1- 2012 283 069

## Description

### Field of the invention

The present invention relates to a tool for exercising the muscles of the mouth, especially a tool for the treatment or relief of symptoms such as secondary tinnitus to disorders of the temporomandibular joint; parafunctional disorders of the jaw with increased muscle work (bruxism and teeth clenching); Secondary dental tension-type headaches; mandibular muscle block (mandibular trismus); non otologic reflected otalgia, atypical facial neuralgia; disck/condyle incoordination of the temporomandibular joint.

### Description of the prior art

At present many devices are known and designed to allow a mandibular exercise.

A first example is described in US1363534, where two simple levers are pivoted and an elastic means provides the necessary resistance. Another example is US-4,280,696 A. From US 3813096 is known a system equipped with a regulation system formed by positioning of a rubber band, which, however, influence the geometrical characteristics of the instrument and in particular the coupling with the oral cavity.

From US4280696 is known a tool for the mandibular exercise in which the counteracting elastic element is obtained from the curvature of the metal support (extension spring).

Finally, in US5035420 is described a tool for the mandibular exercise, provided with a mechanism with a desmodromic system capable of guiding the movable elements of the Tool according to a determined law of motion, in order to perform the stretching exercises of the mandibular joint.

The known solutions, however, are found to be generally unsatisfactory for the contemporary satisfaction of the minimum functions required for the proper mandibular exercise, such as the transfer of a sufficient elastic force modulated in time, the suitability for intra oral contact and the adaptability to the physiology of different patients in terms of opening of the bite and symmetry.

The conditions of maximum efficiency of the mandibular muscles exercise, in order to cure or for the relief of the disorders mentioned above, only occur if the instrument adopted is capable of ensuring the optimal force for the individual patient, variable from person to person and this force is optimally applied for the whole of the movement range, hence coupled at the same time with the capacity to adapt to the physiology of patient himself.

### Object of the invention

Is therefore felt the need for an intraoral mandibular exercise tool, modular and adjustable, able to stretch the inner and outer mandibular muscles so to adapt to the physiology and to the needs of the person, being able to adjust intensity and limits of the mandibular opening.

### Summary of the invention

These purposes have been achieved with an apparatus according to one or more of the attached claims.

A first advantage consists in the fact that the instrument allows people affected by acute and chronic pain of the head and neck the execution of muscle exercises at home, in addition to or instead of expensive professional therapies.

A further advantage consists in the fact that the instrument of the invention is highly efficient but relatively cheap compared to the solutions already known and available in the market.

A further advantage consists in the fact that the instrument of the invention allows the asymmetric adjustment to adapt to different possible configurations of the oral cavity of patients.

A further advantage consists in the fact that the instrument of the invention allows the adjustment of both the amplitude and the symmetry of the bite, regardless of the adjustment of the spring load, and thus allows to perform the exercises in the best way according to both the conformation of the oral cavity and the resistant force, measures only depending on the patient's physiology and the therapeutic choices.

A further advantage consists in the fact that the parts intended for the contact with the mouth of the patient are made in materials suitable for easy and fast periodical sterilization such as stainless steel and food-grade silicone.

### Brief description of the drawings

These and other advantages will be better understood by anyone skilled in the art from the description which follows and from the accompanying drawings given as non-limiting example, in which:

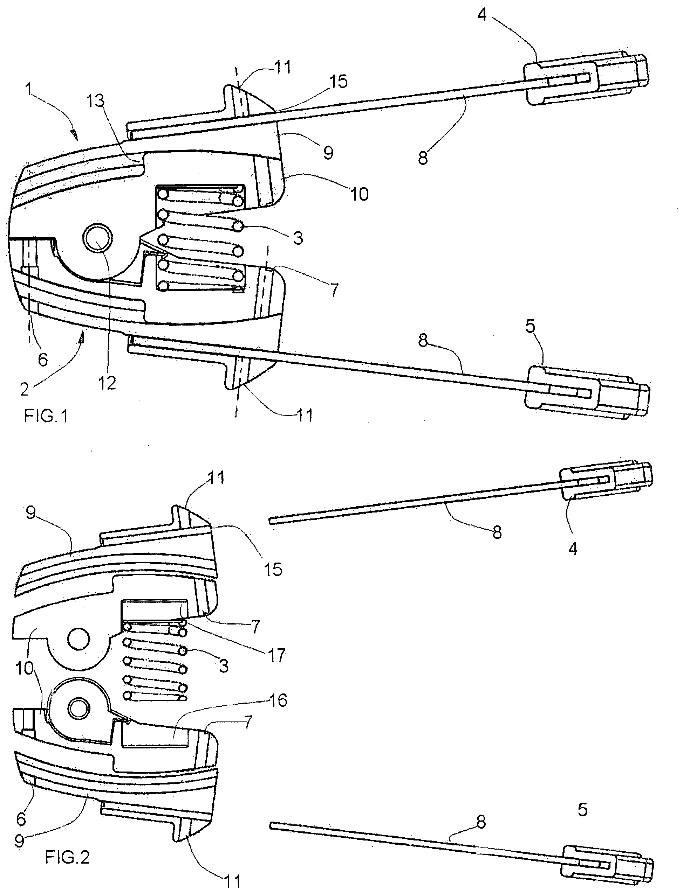

- Fig.1 shows a side view of a preferred embodiment of the instrument;
- Figure 2 shows the instrument of Figure 1 in exploded view;
- Figure 3 shows the tool of Figure 1 in an exploded view, in perspective view;
- The fig.4a, 4b show the tool of Figure 1 in side view with two different settings;
- Figure 5 shows a second embodiment of the device;
- Figure 6 shows a second possible configuration of the device assembly.

### Detailed description of some exemplary embodiments

With reference to the accompanying drawings a preferred embodiment of an instrument for exercising the muscles of the mouth according to the invention is described.

The instrument includes a first and a second body 1, 2 which are mobile with respect to one each other so as to pass from a resting position to at least one operating position against the action of a counteracting elastic element 3.

Preferably, the first and second bodies 1, 2 can turn around a common pin 12 and the elastic element 3 is in the form of a compression element, such as a mechanical spring, pneumatic spring, or a resilient material block, interposed between locations 17, 16 of the first and second bodies 1, 2.

Connected to bodies 1,2 a first and a second portion 4, 5, which are shaped to adapt to gripping with the arches of the upper and lower jaws of a patient, and are arranged in cantilever fashion with respect to said first and second bodies 1, 2, so as to pass from a position at rest where they are away from one another to at least one operating position where they are close to at one another, which correspond to said resting position and said operating position of the bodies 1, 2.

Preferably, the portions 4, 5 are made of food-grade silicone and are connected to bodies 1, 2 by means of movable stainless steel rods 8, sliding in corresponding seats 15 of the bodies 1, 2 to be independently adjusted in a cantilever position along a direction "s" away from the bodies 1, 2 and fixed for example by means of screws pressure 11.

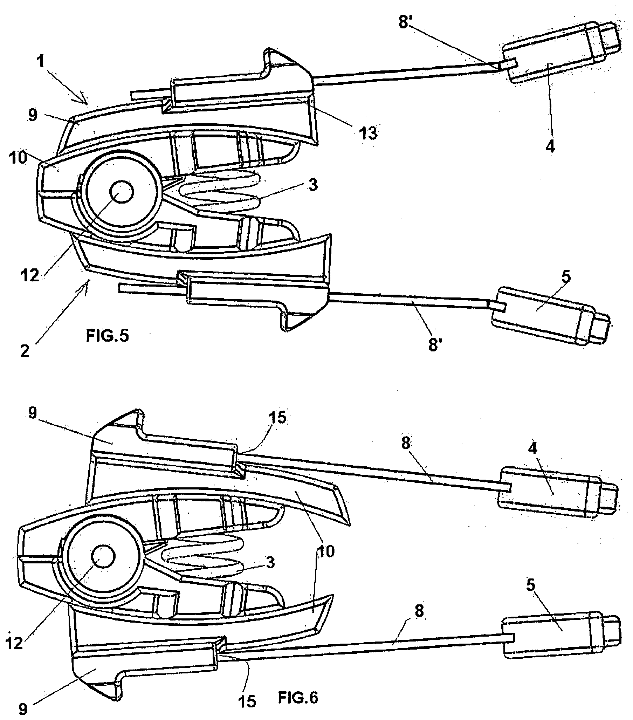

Fig.5 shows a further embodiment of the rods 8, with the terminal parts corresponding to the portions 4, 5 bent in a divergent way in order to facilitate the insertion of the portions 4, 5 into the user's mouth.

The instrument also includes means 6 for adjusting the elastic preloading of the spring 3 at the rest position of the bodies 1 and 2, preferably comprising an adjustable pressure screw 6 between the first and second body to determine a position of contrast at rest.

According to the invention, means 7 are provided for adjusting the initial distance of the portions 4, 5 along a line of approach "a", in the resting position where said portions are away from one another, that are mechanically decoupled with respect to means for adjusting the preloading in order to enable independent adjustment of the distance with respect to the preload.

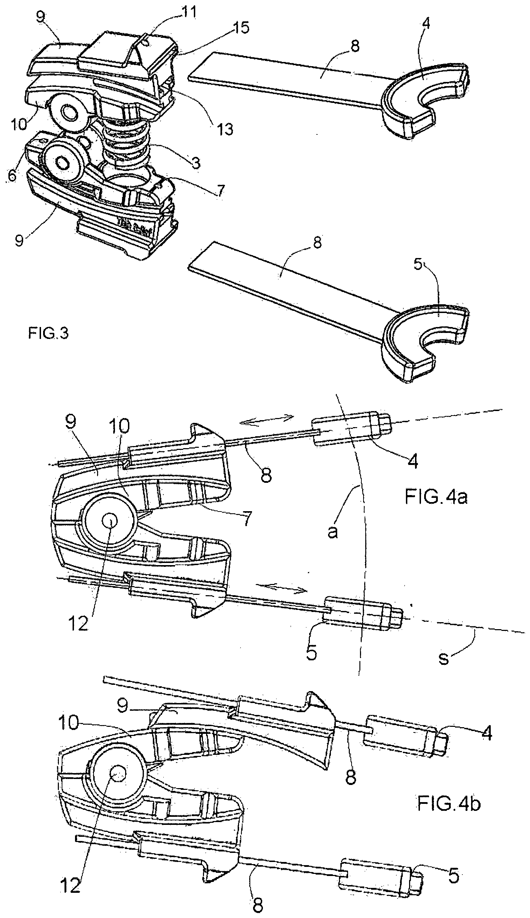

In the described embodiment, the bodies 1, 2 comprise two sectors 9, 10 that are able to slide with respect to one another, by means of a curved-prismatic-guide coupling 13, preferably a circular curve, and the means 7 for adjusting the amplitude of the distance at rest of the portions 4, 5 comprise a pressure screw 7 to adjust and fix the mutual position of the two sectors 9, 10, and then the relative distance of the portions 4, 5 that depends from the point of setting and the curvature of the guide 13 (Figures 4a, 4b).

Advantageously, the shape of the guide 13, for example the circular shape, allows the removal and the reverse fitting, with respect to one another (Figure 6) of the two sectors 9, 10, in order to allow an even greater number of possible mutual positions of the portions 4, 5 and thus increasing the adaptability of the device to different conformations of the user's mouth.

In use, and with reference to the described example, the user can adjust according to the therapy provided the optimum level of preload, acting on the screw 6 of the spring 3 preload.

Moreover, according to the required therapy and / or based on its physiological conformation, the user can adjust the distance at rest of the portions 4, 5 by adjusting the relative position of the sectors 9, 10, without influencing the desired preload.

Alternatively, or in combination, the relative position at rest of the portions 4, 5 can also be adjusted independently of the preload element 3 by sliding more or less the rods 8 through the seats 15, having so one to diverge more or less than the other one.

In addition, fixed the overall preload values and the initial distance of the portions 4, 5, the instrument allows to compensate for the possible asymmetry of the user's mandibular arches, acting on the rods 8 and the screws 11 that determine the cantilevered position of each of the portions 4, 5.

The present invention has been described according to preferred embodiments, various modification in design can be made without departing from the scope of protection of the invention.

## Claims

1. An instrument for exercising the muscles of the mouth, comprising:
a first body (1) and a second body (2), which are mobile with respect to one another so as to pass from a resting position to at least one operating position against the action of a counteracting elastic element (3);
a first portion (4) and a second portion (5), which are shaped to adapt to gripping with the arches of the upper and lower jaws of a patient, and are arranged in cantilever fashion with respect to said first and second bodies (1, 2), respectively, so as to pass from a position at rest where they are away from one another to at least one operating position where they are close to one another, which correspond to said resting position and said operating position of the bodies (1, 2);
and means (6) for adjusting the elastic pre-loading of said elastic element (3) in the resting position of said first and second bodies (1, 2), means (7) for adjusting the distance at rest of the portions (4, 5) along a line of approach (a), in the resting position where said portions (4, 5) are away from one another, said instrument being **characterized in that** said means (6) for adjusting the elastic pre-loading and said means (7) for adjusting the relative distance at rest of the portions (4, 5) are mechanically decoupled to enable independent adjustment of the distance with respect to the pre-load.

2. The instrument according to Claim 1, wherein at least one of said first and second portions (4, 5) is fixed to one of said first and second bodies (1) by cantilever connection means (8, 11) of adjustable length.

3. The instrument according to Claim 1 or Claim 2, wherein said first and second portions (4, 5) are fixed, respectively, to said first and second bodies (1) by cantilever connection means (8, 11) of lengths that can be adjusted independently of one another.

4. The instrument according to any one of the preceding claims, wherein at least one of said first and second bodies (1, 2) comprises two sectors (9, 10) that are able to slide with respect to one another, and said means (7) for adjusting the distance between the portions (4, 5) comprise means for adjustably fixing the mutual position of said sectors (9, 10).

5. The instrument according to Claim 4, wherein said sectors (9, 10) are able to slide by means of a curved-prismatic-guide coupling (13) and can be fixed in position by means of a pressure screw (7).

6. The instrument according to any one of the preceding Claims 2-5, wherein said cantilever connection means (8, 11) of adjustable length of the portions (4, 5) comprise at least one connection rod (8) that can slide with respect to one of said bodies (1, 2).

7. The instrument according to Claim 6, wherein said connection rod (8) is a rod that can slide in a corresponding seat (15) of one of said bodies (1, 2) and can be fixed in position by means of a pressure screw (11).

8. The instrument according to Claim 6, wherein said connection rod (11) is a removable rod.

9. The instrument according to any one of the preceding claims, wherein said first and second bodies (1, 2) can turn about a common pin (12) and said means (6) for adjusting the elastic preloading comprise a pressure screw that acts between said first and second bodies (1, 2) and can be adjusted to determine a position of contrast at rest.

10. The instrument according to any one of the preceding claims, wherein said first and second bodies (1, 2) can turn about a common pin (12), and said elastic element (3) comprises a compression spring set between said first and second bodies (1, 2).

## Patentansprüche

1. Gerät zum Trainieren der Mundmuskulatur, umfassend:
einen ersten Körper (1) und einen zweiten Körper (2), die relativ zueinander beweglich sind, so dass sie von einer Ruheposition zu mindestens einer Betriebsposition gegen die Wirkung eines entgegenwirkenden, elastischen Elements (3) übergehen;
einen ersten Abschnitt (4) und einen zweiten Abschnitt (5), die so geformt sind, dass sie sich zum Ergreifen mit den Bögen des Ober- und Unterkiefers eines Patienten anpassen, und bezüglich des ersten bzw. zweiten Körpers (1, 2) einseitig eingespannt angeordnet sind, so dass sie von einer Ruheposition, in der sie voneinander entfernt sind, zu mindestens einer Betriebsposition, in der sie sich nahe zueinander befinden, übergehen, was der Ruheposition und der Betriebsposition der Körper (1, 2) entspricht;
und Mittel (6) zum Einstellen der elastischen Vorspannung des elastischen Elements (3) in der Ruheposition des ersten und zweiten Körpers (1, 2); Mittel (7) zum Einstellen des Ruheabstands der Abschnitte (4, 5) entlang einer Annäherungslinie (a) in der Ruheposition, bei der die Abschnitte (4, 5) voneinander entfernt sind, wobei das Gerät **dadurch gekennzeichnet ist, dass** die Mittel (6) zum Einstellen der elastischen Vorspannung und die Mittel (7) zum Einstellen des relativen Ruheabstands der Abschnitte (4, 5) mechanisch entkoppelt werden, um eine unabhängige Justierung des Abstands bezüglich der Vorspannung zu ermöglichen.

2. Gerät nach Anspruch 1, wobei mindestens einer des ersten und zweiten Abschnitts (4, 5) an einem des ersten und zweiten Körpers (1) durch einseitig eingespannte Verbindungsmittel (8, 11) von verstellbarer Länge befestigt ist.

3. Gerät nach Anspruch 1 oder Anspruch 2, wobei der erste und zweite Abschnitt (4, 5) jeweils an dem ersten und zweiten Körper (1) durch einseitig eingespannte Verbindungsmittel (8, 11) mit Längen, die unabhängig voneinander eingestellt werden können, befestigt sind.

4. Gerät nach einem der vorhergehenden Ansprüche, wobei mindestens einer des ersten und zweiten Körpers (1, 2) zwei Bogenstücke (9, 10) einschließt, die sich zueinander verschieben können, und die Mittel (7) zum Einstellen des Abstands zwischen den Abschnitten (4, 5) Mittel zur verstellbaren Befestigung der wechselseitigen Position der Bogenstücke (9, 10) umfassen.

5. Gerät nach Anspruch 4, wobei sich die Bogenstücke (9, 10) durch eine Verbindung (13) mit bogenförmiger prismatischer Führung verschieben können und durch eine Druckschraube (7) in Position befestigt werden können.

6. Gerät nach einem der vorhergehenden Ansprüche 2 bis 5, wobei die einseitig eingespannten Verbindungsmittel (8, 11) verstellbarer Länge der Abschnitte (4, 5) mindestens einen Verbindungsstab (8) umfassen, der sich relativ zu einem der Körper (1, 2) verschieben kann.

7. Gerät nach Anspruch 6, wobei der Verbindungsstab (8) ein Stab ist, der sich in einem entsprechenden Sitz (15) von einem der Körper (1, 2) verschieben kann und durch eine Druckschraube (11) in Position befestigt werden kann.

8. Gerät nach Anspruch 6, wobei der Verbindungsstab (8) ein entfernbarer Stab ist.

9. Gerät nach einem der vorhergehenden Ansprüche, wobei sich der erste und zweite Körper (1, 2) um einen gemeinsamen Stift (12) drehen können und die Mittel (6) zur Einstellung der elastischen Vorspannung eine Druckschraube umfassen, die zwischen dem ersten und zweiten Körper (1, 2) wirksam ist und verstellt werden kann, um eine Gegenposition bei Ruhe festzulegen.

10. Gerät nach einem der vorhergehenden Ansprüche, wobei sich der erste und zweite Körper (1, 2) um einen gemeinsamen Stift (12) drehen können, und das elastische Element (3) eine Druckfeder umfasst, die zwischen dem ersten und zweiten Körper (1, 2) eingesetzt ist.

## Revendications

1. Instrument pour exercer les muscles de la bouche, comprenant :
un premier corps (1) et un deuxième corps (2), qui sont mobiles l'un par rapport à l'autre de manière à passer d'une position de repos à au moins une position de fonctionnement contre l'action d'un élément élastique compensateur (3) ;
une première partie (4) et une deuxième partie (5), qui sont profilées pour s'adapter à la prise avec les arcs des mâchoires supérieure et inférieure d'un patient, et sont agencées en porte-à-faux respectivement par rapport auxdits premier et deuxième corps (1, 2), de manière à passer d'une position au repos dans laquelle elles sont éloignées l'une de l'autre à au moins une position de fonctionnement dans laquelle elles sont proches l'une de l'autre, qui correspondent à ladite position de repos et ladite position de fonctionnement des corps (1, 2) ;
et des moyens (6) pour régler la pré-charge élastique dudit élément élastique (3) dans la position de repos desdits premier et deuxième corps (1, 2), des moyens (7) pour régler la distance au repos des parties (4, 5) suivant une ligne d'approche (a), dans la position de repos dans laquelle lesdites parties (4, 5) sont éloignées l'une de l'autre, ledit instrument étant **caractérisé en ce que** lesdits moyens (6) pour régler la pré-charge élastique et lesdits moyens (7) pour régler la distance relative au repos des parties (4, 5)sont découplés mécaniquement pour permettre un réglage indépendant de la distance par rapport à la pré-charge.

2. Instrument selon la revendication 1, dans lequel au moins une desdites première et deuxième parties (4, 5) est fixée à un desdits premier et deuxième corps (1) par des moyens de connexion en porte-à-faux (8, 11) de longueur réglable.

3. Instrument selon la revendication 1 ou la revendication 2, dans lequel lesdites première et deuxième parties (4, 5) sont fixées, respectivement, auxdits premier et deuxième corps (1) par des moyens de connexion en porte-à-faux (8, 11) de longueurs qui peuvent être réglées indépendamment l'une de l'autre.

4. Instrument selon l'une quelconque des revendications précédentes, dans lequel au moins un desdits premier et deuxième corps (1, 2) comprend deux secteurs (9, 10) qui sont adaptés pour coulisser l'un par rapport à l'autre, et lesdits moyens (7) pour régler la distance entre les parties (4, 5) comprennent des moyens pour fixer de manière réglable la position mutuelle desdits secteurs (9, 10).

5. Instrument selon la revendication 4, dans lequel lesdits secteurs (9, 10) sont adaptés pour coulisser au moyen d'un couplage de guide prismatique incurvé (13) et peuvent être fixés en position au moyen d'une vis de pression (7).

6. Instrument selon l'une quelconque des revendications précédentes 2 à 5, dans lequel lesdits moyens de connexion en porte-à-faux (8, 11) de longueur réglable des parties (4, 5) comprennent au moins une tige de connexion (8) qui peut coulisser par rapport à un desdits corps (1, 2).

7. Instrument selon la revendication 6, dans lequel ladite tige de connexion (8) est une tige qui peut coulisser dans un siège correspondant (15) d'un desdits corps (1, 2) et peut être fixée en position au moyen d'une vis de pression (7).

8. Instrument selon la revendication 6, dans lequel ladite tige de connexion (8) est une tige amovible.

9. Instrument selon l'une quelconque des revendications précédentes, dans lequel lesdits premier et deuxième corps (1, 2) peuvent tourner autour d'un axe commun (12) et lesdits moyens (6) pour régler la pré-charge élastique comprennent une vis de pression qui agit entre lesdits premier et deuxième corps (1, 2) et peuvent être réglés pour déterminer une position de contraste au repos.

10. Instrument selon l'une quelconque des revendications précédentes, dans lequel lesdits premier et deuxième corps (1, 2) peuvent tourner autour d'un axe commun (12) et ledit élément élastique (3) comprend un ressort de compression installé entre lesdits premier et deuxième corps (1, 2).
